(19) Europäisches Patentamt
European Patent Office
Office européen des brevets

(11) **EP 2 440 920 B1**

(12) **EUROPÄISCHE PATENTSCHRIFT**

(45) Veröffentlichungstag und Bekanntmachung des Hinweises auf die Patenterteilung:
**10.09.2014 Patentblatt 2014/37**

(21) Anmeldenummer: **10734962.3**

(22) Anmeldetag: **13.07.2010**

(51) Int Cl.:
***G01N 33/483*** *(2006.01)* ***G01N 33/49*** *(2006.01)*
***G06K 9/00*** *(2006.01)*

(86) Internationale Anmeldenummer:
**PCT/EP2010/060082**

(87) Internationale Veröffentlichungsnummer:
**WO 2011/009775 (27.01.2011 Gazette 2011/04)**

(54) **LOKALISIERUNG EINES VALIDEN BEREICHES EINES BLUTAUSSTRICHES**

LOCALIZING A VALID REGION OF A BLOOD SMEAR

LOCALISATION D'UNE RÉGION SAINE D'UN FROTTIS SANGUIN

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **20.07.2009 DE 102009033927**

(43) Veröffentlichungstag der Anmeldung:
**18.04.2012 Patentblatt 2012/16**

(73) Patentinhaber: **Fraunhofer-Gesellschaft zur Förderung der angewandten Forschung e.V.
80686 München (DE)**

(72) Erfinder:
 • **SCHLARB, Timo
 90766 Fürth (DE)**
 • **RUPP, Stephan
 90765 Fürth (DE)**
 • **ZERFASS, Thorsten
 90408 Nürnberg (DE)**

(74) Vertreter: **Schenk, Markus et al
Schoppe, Zimmermann, Stöckeler & Zinkler
Patentanwälte
Postfach 246
82043 Pullach bei München (DE)**

(56) Entgegenhaltungen:
 **DE-A1- 10 353 785 US-A- 4 702 595**

 • **MUES-HINTERWÄLLER ET AL: "Detektion und berandungsgenaue Segmentierung von Erythrozyten" 1. Januar 2005 (2005-01-01), BILDVERARBEITUNG FÜR DIE MEDIZIN, SPRINGER VERLAG, DE, PAGE(S) 1 - 5 , XP007915115 das ganze Dokument**
 • **XIONG W ET AL: "Automatic working area classification in peripheral blood smears without cell central zone extraction" ENGINEERING IN MEDICINE AND BIOLOGY SOCIETY, 2008. EMBS 2008. 30TH ANNUAL INTERNATIONAL CONFERENCE OF THE IEEE, IEEE, PISCATAWAY, NJ, USA, 20. August 2008 (2008-08-20), Seiten 4074-4077, XP031508904 ISBN: 978-1-4244-1814-5**
 • **XIONG W ET AL: "Automatic working area classification in peripheral blood smears using spatial distribution features across scales" 19TH INTERNATIONAL CONFERENCE ON PATTERN RECOGNITION, 2008: ICPR 2008; 8 - 11 DEC. 2008, TAMPA, FLORIDA, USA, IEEE, PISCATAWAY, NJ, 8. Dezember 2008 (2008-12-08), Seiten 1-4, XP031412336 ISBN: 978-1-4244-2174-9**
 • **ANGULO J ET AL: "Automated detection of working area of peripheral blood smears using mathematical morphology" ANALYTICAL CELLULAR PATHOLOGY, ELSEVIER SCIENCE, AMSTERDAM, NL, Bd. 25, 1. Januar 2003 (2003-01-01), Seiten 37-49, XP007915116 ISSN: 0921-8912**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 440 920 B1

**Beschreibung**

Die vorliegende Erfindung bezieht sich auf die Lokalisierung eines validen Bereiches eines Blutausstriches, wie z.B. auf einem Objektträger.

Verfahren für die computerassistierte Diagnose (CAD) unterstützen den Arzt in seiner Diagnöseentscheidung. Ziel dieser Verfahren ist einerseits die Qualität der Diagnosen zu verbessern und andererseits kostspielige Doppelbefundungen durch einen zweiten Arzt zu ersetzen. In den letzten Jahren wurden CAD Systeme unter anderem für die Diagnose von Hautkrebs, Brustkrebs und zur Diagnose des Blutbildes entwickelt.

Für die medizinische Diagnostik ist Blut ein unerlässlicher Indikator einer großen Bandbreite an Erkrankungen, d.h. für über Blut, über Parasiten und sexuell übertragbare Krankheiten. Für routinemäßige Untersuchungen wird das Blut üblicherweise mittels Durchflusszytometrie oder die Verwendung einer fluoreszenzaktivierten Zellsortierung (FACS - fluorescence activated cell sorting) automatisch analysiert. Ungünstigerweise liefern diese automatischen Verfahren in 30 % bis 40 % der Analysen keine zufrieden stellenden Ergebnisse. Deshalb ist in diesen Fällen die anschließende visuelle Prüfung des Blutes von essentieller Bedeutung. Außerdem gibt es viele Anwendungsfälle, bei denen eine visuelle Prüfung eines Blutausstrichs erforderlich ist, auch wenn ein pathologischer Verdacht besteht.

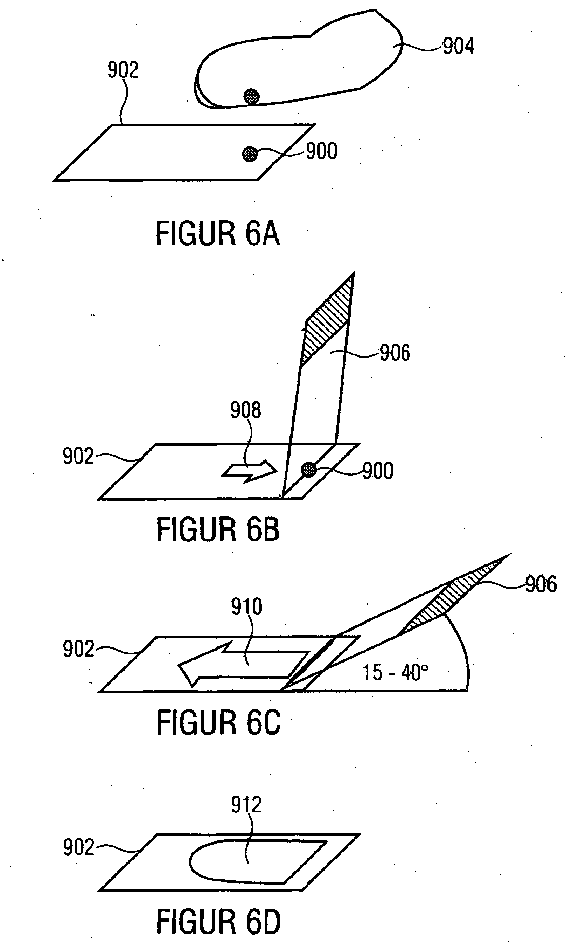

Mit "Blutausstrich" wird ein Verfahren zur Vorbereitung von Blut für beispielsweise eine mikroskopische Untersuchung bzw. das Ergebnis dieses Verfahrens bezeichnet. Den Vorgang des Anfertigens eines Blutausstriches zeigt exemplarisch Fig. 6. Blutausstriche werden beispielsweise für zytologische und hämatologische Untersuchungen von Blutzellen, wie z.B. zur Zählung der weißen Blutkörperchen bzw. Blutzellen, und zum Nachweis der Existenz von Bakterien oder Parasiten im Blut, wie z.B. Malaria, verwendet.

Wie es in Fig. 6 gezeigt ist, beginnt der Vorgang des Anfertigens eines Blutausstriches mit dem Aufbringen eines Blutstropfens 900 auf einem Objektträger 902, beispielsweise durch einen Fingerpieks, wie es durch den Finger 904 angedeutet ist (vgl. Fig. 6a). Der Blutstropfen 900 wird insbesondere in der Nähe einer der Schmalseiten des Objektträgers 902 aufgebracht. Sodann wird ein zweiter Objektträger 906 oder ein ähnlicher plattenförmiger Gegenstand mit seiner Kante auf den Objektträger 900 aufgesetzt, und zwar in demjenigen Teil des Objektträgers 902, der der schmalen Seite desselben, in deren Nähe sich der Blutstropfen 900 befindet, über den Blutstropfen 900 gegenüberliegt. Dann wird der in Richtung Blut geneigte Objektträger 906 vorsichtig an den Blutstropfen 900 herangeführt, wie es mit dem Pfeil 908 angedeutet ist, bis der Objektträger 906 Kontakt mit dem Blutstropfen 900 erhält und das Blut sich seitlich entlang der Kante des Objektträgers 906 zu verteilen beginnt (vgl. Fig. 6b). Dann wird der Objektträger 906 weiter in Richtung entgegen der Ausstrichrichtung 910 geneigt, wie z.B. auf 15° bis 40°, wie es in Fig. 6c gezeigt ist. Dann wird in Ausstrichrichtung 910 weg von dem nahegelegenen schmalen Rand des Objektträgers 902 das Blut auf dem Objektträger 902 verteilt, indem der Objektträger 906 unter dem genannten Winkel über den Objektträger 902 gestreift wird, das Blut quasi im "Schlepp". Das Ergebnis ist in Fig. 6d gezeigt: der Blutausstrich 912 auf dem Objektträger 902. Der Blutausstrich 912 ist somit ein dünner Film von Blut auf dem Objektträger 902. Er wird getrocknet und eventuell einer Färbung unterzogen, wie z.B. einer Brehmer-Färbung oder dergleichen. Eventuell wird auch eine Fixierung mittels beispielsweise Methanol vorgenommen. Vgl. THEML, H., H. OIEM und T. HAFERLACH: Taschenatlas der Hämatologie. Thiem Verlag, Stuttgart, New York, 2002" (Seiten 16 - 18, Kapitel: 'Der Blutausstrich und seine Auswertung').

In anderen Worten ausgedrückt wird, um einen Blutausstrich zu erhalten, eine kleine Menge Blut an einem Ende eines Mikroskopobjektträgers platziert und entlang von dessen voller Länge verteilt. Das Ziel dabei ist, eine Region zu erhalten, wo die Blutzellen weit genug voneinander entfernt sind, um gezielt und differenziert bzw. klassifiziert zu werden. Diese Region wird häufig als die valide Region oder der Arbeitsbereich bezeichnet. Die Anfertigung eines solchen Blutausstriches kann automatisch oder manuell erfolgen. Unglücklicherweise sind Blutausstricherzeugungsgeräte sehr teuer und resultieren somit in hohen Kosten. Die Folge ist, dass Labore kleiner oder mittlerer Größe auf eine manuelle Aus-/Anfertigung zurückgreifen müssen. Es sind aber eine hohe Fertigkeit und Erfahrung durch den Laborassistenten notwendig, um einen guten Blutausstrich mit einer validen Region zu erzielen, die groß genug und geeignet für die Untersuchung ist. Und trotzdem noch ist-eine geeignete Blutausstrichpräparation nicht garantiert und muss immer noch häufig wiederholt werden. Wie bereits erwähnt, kann der Blutausstrich nach seiner Anfertigung vollständig luftgetrocknet und der Objektträger mit Methanol benetzt werden, um den Blutausstrich zu fixieren, woraufhin eine Färbung vorgenommen werden kann, um die Blutzellen sichtbar zu machen.

Am Fraunhofer IIS wurde mit dem System HemaCAM® für die Hämatologie ein computerassistiertes Diagnosesystem zur automatischen Analyse von Blutausstrichen zur Erstellung eines Differentialblutbildes entwickelt (vgl. Mues-Hinterwäller, S., Kuziela, H., Grobe M. Wittenberg, T.: Detektion und berandungsgenaue Segmentierung von Erythrozyten. Bildverarbeitung für die Medizin(2005)380-384).

Die sichere Erkennung und exakte Segmentierung von weißen Blutzellen (Leukozyten) in gefärbten Ausstrichen des peripheren Blutes bildet die Grundlage für eine automatische, bildbasierte Erstellung eines sog. Differentialblutbildes im Kontext der medizinischen Labordiagnostik (sog. Computer Assistierte Mikroskopie - CAM). Die Vielfältigkeit der in einem Blutausstrich auftretenden weißen Blutzellen, verbunden mit ihrer jeweils charakteristischen Farbverteilung und Texturierung, erhöhen die Schwierigkeit bei der Klassifikation im Rahmen einer vollständigen Automatisierung. Die automatische Detektion und Segmentierung weißer Blutzellen in digitalen Bildern ermöglicht die anschließende berandungsgenaue Segmentierung von Zellkern und Zellplasma im Hinblick auf eine nachfolgende Klassifikation. Voraussetzung für die Detektion und Segmentierung und damit entscheidend für die Aussagekraft der Diagnose ist, dass die Blutzellen auf dem Objektträger im validen Bereich ausgewertet werden.

Der valide Bereich auf dem Objektträger ist dadurch gekennzeichnet, dass die Blutzellen nebeneinander, nicht übereinander und in fast gleichen Abständen zueinander liegen. Die Blutzellen berühren sich nur vereinzelt; sie bilden keine Zellketten und keine Zellhaufen. Nach THEML, H., H. DIEM und T. HAFERLACH: Taschenatlas der Hämatologie, Thieme Verlag, Stuttgart, New York, 2002 befindet sich dieser Bereich erfahrungsgemäß etwa 1 cm hinter dem Bartende. Bei einem Ausstrich mit normalen Leukozytenwerten befinden sich beim Betrachten mit einer 40x Objektvergrößerung durchschnittlich zwei bis drei Leukozyten pro Blickfeld.

Wünschenswert wäre somit ein Schema zur Lokalisierung eines validen Bereichs eines Blutausstriches zu besitzen, der eine automatische Lokalisierung ermöglicht, so dass der Untersuchungsaufwand gesenkt und die Untersuchungsergebnisse vergleichbarer gemacht werden können, da sie nach benutzerunabhängigen bzw. objektiven Kriterien erhalten werden.

Die DE 103 53 785 B4 beschreibt ein Verfahren und eine Vorrichtung zur Erfassung von verschiedenen Zelltypen von Zellen in einer biologischen Probe, und insbesondere die automatische Erstellung eines Differenzial-Blutbildes basierend auf digitalisierten mikroskopischen Aufnahmen von Blutausstrichen mit Hilfe von Bildverarbeitungsverfahren. Zur Digitalisierung der Blutprobe wird das Objektiv mäanderformig relativ zur Blutprobe bewegt. Die Einzelbilder werden dann einer Bildverarbeitung zugeführt, um die Zellen zu klassifizieren und zu zählen, um somit ein Differenzialblutbild zu erstellen.

Die Aufgabe der vorliegenden Erfindung besteht deshalb darin, ein Schema zur Lokalisierung eines validen Bereichs eines Blutausstriches zu schaffen, der dies ermöglicht.

Diese Aufgabe wird durch eine Vorrichtung gemäß Anspruch 1 und ein Verfahren gemäß Anspruch 12 gelöst.

Ein Kerngedanke der vorliegenden Erfindung besteht darin, dass eine automatisierte Lokalisierung eines validen Bereichs eines Blutausstriches und damit eine unaufwendigere und objektivere Lokalisierung ermöglicht wird, wenn in einer Aufnahme des Blutausstriches Bildpunkte zumindest in erste Bildpunkte, die Blutzellen darstellen, und zweite Bildpunkte, die die Blutzellen nicht darstellen, klassifiziert werden, und dann der valide Bereich auf Basis einer lokalen Häufigkeit von Bildpunktclustern von zumindest $A_{min}$ ersten Bildpunkten, wobei $A_{min}$ ein Mindestschwellwert für eine Anzahl von ersten Bildpunkten eines Bildpunktclusters ist, und einer lokalen mittleren Größe der Bildpunktcluster für lateral verteilte Bereiche des Blutausstriches getroffen wird.

Gemäß einem Ausführungsbeispiel der vorliegenden Erfindung wird auf Basis der lokalen Häufigkeiten und lokalen mittleren Größen der Bildpunktcluster für die lateral verteilten Bereiche ein lateral variierendes Maß für eine Eignung als valider Bereich bestimmt, wobei das lateral variierende Maß von der lokalen Häufigkeit und von der lokalen mittleren Größe gemäß seiner Funktion abhängt, die entweder streng monoton fallend für die lokale Häufigkeit und streng monoton steigend für die lokale mittlere Größe oder streng monoton steigend für die lokale Häufigkeit und streng monoton fallend für die lokale mittlere Größe ist, wobei als valider Bereich dann derjenige Bereich der lateral verteilten Bereiche ausgewählt wird, bei welchem das lateral variierende Maß für die Eignung als valider Bereich extremal ist.

Bevorzugte Ausführungsbeispiele der vorliegenden Erfindung werden nachfolgend Bezug nehmend auf die beiliegenden Zeichnungen näher erläutert. Es zeigen:

Fig. 1 ein Blockschaltbild einer Vorrichtung zur Lokalisierung eines validen Bereiches eines Blutausstriches gemäß einem Ausführungsbeispiel;

Fig. 2 eine schematische Zeichnung zur Veranschaulichung einer Lokalisierung eines validen Bereiches eines Blutausstriches wie z.B. durch die Vorrichtung von Fig. 1 gemäß einem Ausführungsbeispiel;

Fig. 3a-c exemplarische Aufnahmen unterschiedlicher Bereiche eines Blutausstriches, wobei Fig. 3a eine geringe Zelldichte aufweist, Fig. 3b einem validen Bereich entspricht und Fig. 3c eine hohe Zelldichte aufweist;

Fig. 4a-c Darstellung der Aufnahmen aus Fig. 3a-3c nach erfolgter Klassifizierung der Bildpunkte nach Vordergrund bzw. Blutzellen und Hintergrund .gemäß einem Ausführungsbeispiel;

Fig. 5 einen Graphen, in welchem für eine exemplarische Abtastung eines Blutausstriches mit 36 Aufnahmen an in Ausstrichrichtung zueinander versetzten Bereichen des Blutausstriches die laterale Verteilung der lokalen Häufigkeit, der lokalen mittleren Größe und ein Maß für die Eignung als valider Bereich gemäß zweier Ausführungsbeispiele aufgetragen ist; und

Fig. 6a-d schematische Zeichnungen zur Veranschaulichung möglicher Verfahrensschritte zur Anfertigung eines Blutausstriches.

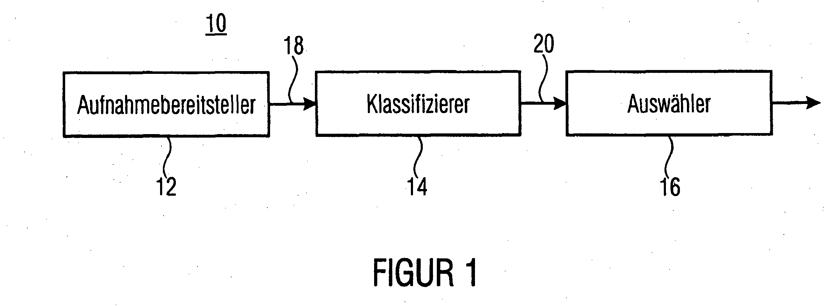

Fig. 1 zeigt eine Vorrichtung zur Lokalisierung eines validen Bereiches eines Blutausstriches. Die Vorrichtung ist allgemein mit 10 angezeigt. Sie umfasst einen Aufnahmebereitsteller 12, einen Klassifizierer 14 und einen Auswähler 16.

Der Aufnahmebereitsteller 12 stellt zumindest eine Aufnahme des Blutausstriches bereit. Vorzugsweise deckt die zumindest eine Aufnahme den Blutausstriche vollständig ab. Bei den nachfolgend beschriebenen Ausführungsbeispielen stellt der Aufnahmebefeitsteller 12 mehrere Aufnahmen des Blutausstriches bereit, die jeweils nur einen Teil des Blutausstriches darstellen, wobei die Teile lateral zueinander versetzt sind. Sie können sich bereichsmäßig überlappen, aneinandergrenzen oder auch voneinander beabstandet sein. Die Aufnahmen sind beispielsweise mit einer 10-fachen, 20-fachen oder einer dazwischenliegenden Vergrößerung erhalten worden. Dabei kann der Aufnalunebereitsteller 12 so ausgebildet sein, dass diese Aufnahmen eine Auflösung von 400x400 Bildpunkten, 2000x2000 Bildpunkten oder eine dazwischenliegenden Auflösung aufweisen.

Der Aufnahmebereitsteller 12 kann beispielsweise einen Datenspeicher 12 aufweisen, wie z.B. einen nichtflüchtigen Speicher, wie z.B. eine Festplatte oder ein Flash-Speicher oder dergleichen. In diesem Speicher sind die Aufnahmen xlann vorab eingespeichert worden, wie z.B. direkt nach ihrer Erstellung. Der Aufhahmebereitsteller 12 kann allerdings auch eine Aufnahmevorrichtung, wie z.B. ein Mikroskop mit einem Pixelarray, aufweisen, um die zumindest eine Aufnahme anzufertigen.

Die Ausgabe des Aufnahmebereitstellers 12 umfasst also zumindest eine Aufnahme 18 des Fluidausstriches, und der Klassifizierer 14 ist ausgebildet, um an dieser zumindest einen Aufnahme 18 eine Klassifikation der Bildpunkte vorzunehmen. Insbesondere ist der Klassifizierer 14 dazu ausgebildet, die Bildpunkte der zumindest einen Aufnahme zumindest in erste Bildpunkte, die Blutzellen darstellen, und zweite Bildpunkte, die die Blutzellen nicht darstellen, zu klassifizieren bzw. einzuteilen. Das Ergebnis der Klassifikation kann also beispielsweise ein Binärbild oder eine Mehrzahl von Binärbildern sein, das bzw. die aus der zumindest einen Aufnahme 18 jeweils entsteht. Der Klassifizierer 14 kann so ausgebildet sein, dass er nicht zwischen weißen und roten Blutzellen unterscheidet. Gemäß einem Ausführungsbeispiel, das im Folgenden noch erläutert wird, kann es aber auch sein, dass der Klassifizierer 14 zwischen roten und weißen Blutzellen unterscheidet und die Bildpunkte in erste Bildpunkte, die die roten Blutzellen darstellen, und zweite Bildpunkte, die die roten Blutzellen nicht darstellen, klassifiziert. Die Klassifikation führt der Klassifizierer beispielweise durch Unterteilung eines Farbraumes in unterschiedliche, den einzelnen Bildpunktklassen entsprechende Bereiche und Klassifizieren der Bildpunkte abhängig von der Lage des Farbwertes der einzelnen Bildpunkte in einem der Teile durch. Später wird dies noch deutlicher ausgeführt.

Das Ergebnis der Klassifikation durch den Klassifizierer 14 ist also eine nach Blutzellen-bildpunkten und Nicht-Blutzellen-Bildpunkten klassifizierte Version 20 der zumindest einen Aufnahme 18 des Blutausstriches. Der Auswähler 16 ist ausgebildet, um auf Basis dieser klassifizierten Version 20 den validen Bereich auszuwählen. Klassifizierer 14 und Auswähler 16 können beispielsweise beide durch Software auf einem Prozessor implementiert sein.

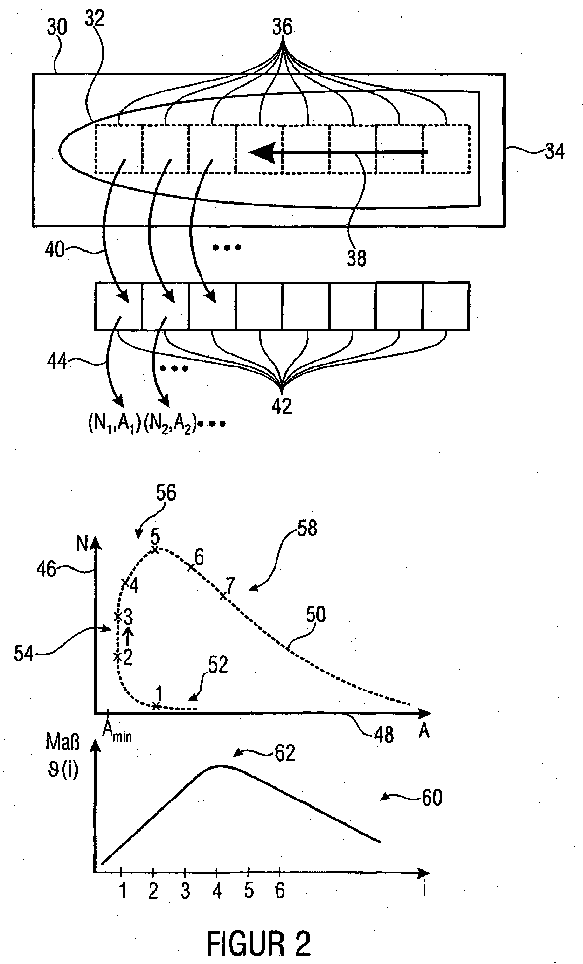

Der Auswähler 16 ist ausgebildet, um die Auswahl des validen Bereiches auf Basis einer lokalen Häufigkeit von zusammenhängenden Bildpunktclustern von zumindest $A_{min}$ ersten Bildpunkten, wobei $A_{min}$ ein Mindestschwellwert für eine Anzahl von ersten Bildpunkten eines Bildpunktclusters ist, und einer lokalen mittleren Größe der Bildpunktcluster für lateral verteilte Bereiche des Blutausstriches zu treffen. Vorzugsweise sind die lateral verteilten Bereiche des Blutausstriches entlang der Blutausstrichrichtung des Blutausstriches verteilt. Gemäß Ausführungsbeispielen, die im Folgenden beschrieben werden, entsprechen die lateral verteilten Bereiche jeweils einer Aufnahme 18, wobei die lokale Häufigkeit dann der Anzahl der Bildpunktcluster in einer jeweiligen Aufnahme, und die lokale mittlere Größe der mittleren Größe der Bildpunktcluster in einer jeweiligen Aufnahme entspricht. Wie es ferner im Folgenden beschrieben wird, kann der Auswähler 16 so ausgebildet sein, dass er die Auswahl des validen Bereiches mittels eines Maßes bzw. einer skalaren Größe bestimmt, das bzw. die ein zumindest ordinal skaliertes Maß für eine Eignung als valider Bereich darstellt. Dieses Maß bzw. diese skalierbare Variable kann von der lokalen Häufigkeit und der lokalen mittleren Größe gemäß einer Funktion abhängen, die entweder streng monoton fallend für die lokale Häufigkeit und streng monoton steigend für die lokale mittlere Größe oder streng monoton steigend für die lokale Häufigkeit und streng monoton fallend für die lokale mittlere Größe ist. Im Folgenden werden konkrete Beispiele hierfür geliefert. Es kann allerdings auch sein, dass der Auswähler 16 die Auswahl des validen Bereiches auf andere Weise auf Basis der lokalen Häufigkeit und der lokalen mittleren Größe ermittelt, wie z.B. auf Basis einer Auswertung der Spur, entlang der die Tupel aus lokaler Häufigkeit und lokaler mittlerer Größe für die lateral verteilten Bereiche in einer Ebene liegen, die durch eine Achse für die lokale Häufigkeit und eine Achse für die lokale mittlere Größe aufgespannt wird. Die verschiedenen Alternativen werden anhand von Fig. 2 näher veranschaulicht.

Fig. 2 stellt eine mögliche Vorgehensweise bei der Lokalisierung eines validen Bereichs eines Blutausstriches dar, wie sie exemplarisch durch die Vorrichtung von Fig. 1 vornenommen werden kann. Fig. 2 zeigt einen Objektträger 30 mit einem Blutausstrich 32 auf einer Oberfläche 34 desselben. Gemäß Fig. 2, stellt der Aufnahmebereitsteller 12 mehrere Aufnahmen 36 des Blutausstriches 32 bereit bzw. erstellt dieselben. Wie es in Fig. 2 gezeigt ist, decken die Aufnahmen 36 unterschiedliche Teile des Blutausstriches 32 ab. In Fig. 2 sind sie zwar exemplarisch so dargestellt, als ob sie nicht-überlappende aber dafür aneinander grenzende Teile des Blutausstriches 32 darstellen, aber die Aufnahmen 36 können sich ebenso überlappen und/oder sie können ebenso voneinander beabstandet sein. Wie es in Fig. 2 gezeigt ist, können die Aufnahmen 36 in Blutausstrichrichtung 38 versetzt zueinander angeordnet sein. Es ist aber auch möglich, dass die Aufnahmen 36 zumindest teilweise auch in Blutausstrichrichtung 38 nebeneinander liegen. Im Folgenden wird davon ausgegangen, dass die Aufnahmen 36 in Blutausstrichrichtung 38 so versetzt zueinander angeordnet sind, dass sie bezogen auf die Blutausstrichrichtung 38 eindeutig in eine Reihenfolge gebracht werden können, wobei sich im Folgenden die Indizes, die die Aufnahmenummer angeben, auf diese Reihenfolge beziehen sollen. Den Aufnahmen sind die Orte auf bzw. entlang des Blutausstriches zugeordnet, an denen der Bereich angeordnet ist, den die jeweilige Aufnahme von dem Blutausstrich zeigt. Beispielsweise werden bestimmte Orte von einem Verfahrgerät angefahren, so dass die Aufnahmen an diesen Orten aufgenommen werden. Oder die Orte werden bei Aufnahme gemessen, ohne dass sie zur Regelung oder Steuerung der Relativbewegung zwischen Aufnahmeersteller bzw. Mikroskop und Objektträger verwendet werden.

Wie es mit Pfeilen 40 angedeutet ist, werden dann die Bildpunkte in den einzelnen Aufnahmen 36 klassifiziert, wie z.B. durch den Klassifizierer 14 von Fig. 1. Die Klassifikation kann, wie im Vorhergehenden erwähnt, zwischen ersten Bildpunkten, die Blutzellen darstellen, und zweiten Bildpunkten, die die Blutzellen nicht darstellen, unterscheiden. Das Ergebnis sind klassifizierte Versionen 42 der Aufnahmen 36, auf denen die Bildpunkte, die die interessierenden Blutzellen darstellen, von den anderen Bildpunkten, die diese Blutzellen nicht darstellen, unterscheidbar sind. Bei diesen klassifizierten Versionen 42 kann es sich beispielsweise um Binärbilder handeln. Bei den interessierenden Blutzellen kann es sich beispielsweise um lediglich die weißen Blutzellen handeln, um lediglich die roten Blutzellen oder um rote und weiße Blutzellen. Zur leichteren Klassifikation ist der Blutausstrich 32 beispielsweise vor der Erstellung der Aufnahmen 36 einem geeigneten Färbeverfahren unterzogen worden, wofür im Folgenden noch ein Ausführungsbeispiel erwähnt wird. Die Klassifikation kann beispielsweise basierend auf einer Aufteilung eines Farbraums, wie z.B. des HSV-Farbraums, in einen ersten Teil und einen zweiten Teil durchgeführt werden. Bildpunkte, deren Farbwert im ersten Teil liegt, werden dann beispielsweise als eine interessierende Blutzelle darstellend interpretiert, und Bildpunkte, deren Farbwerk im zweiten Teil liegt, werden als keine interessierende Blutzelle darstellend interpretiert.

Wie es mit Pfeilen 44 angedeutet ist, wird zu jedem der Bilder 42 ein Tupel bzw. -ein Paar von Werten bestimmt, nämlich eine lokale Häufigkeit bzw. eine lokale Anzahl von Bildpunktclustern aus solchen Bildpunkten in dem jeweiligen Bild, die eine interessierende Blutzelle darstellen, und eine mittlere Größe dieser Bildpunktcluster. Auf diese Weise entstehen, wie es in Fig. 2 gezeigt ist, für jedes Bild i der Bilder 42 zwei Werte, nämlich eine lokale Häufigkeit $N_i$ und eine lokale mittlere Größe $A_i$. Im Folgenden wird noch ein konkretes Ausführungsbeispiel für die Bestimmung von $N_i$ und $A_i$ geliefert. Auf jeden Fall kann es sein, dass der Auswähler 16 bei der Bestimmung 44 nur die zusammenhängenden Bildpunktcluster zählt, die aus einer Anzahl von aneinanderangrenzenden Blutzellen-Bildpunkten bestehen, die eine vorbestimmte Mindestanzahl $A_{min}$ überschreitet. Die lokale mittlere Größe $A_i$ wird beispielsweise von dem Auswähler 16 nur für diese Bildpunktcluster bestimmt. Als Maß für die Größe wird beispielsweise die Anzahl der Bildpunkte eines Bildpunktclusters verwendet. Zur Mittelung über die Häufgkeitsverteilung der Größen der Bildpunktcluster in dem jeweiligen lokalen Bereich bzw. Bild 42 kann jegliche zentrale Tendenz herangezogen werden, wie z.B. der Mittelwert, der Modus oder der Median. Die Aufnahmen werden beispielsweise mit einer 10-fachen, einer 20-fachen oder einer dazwischen liegenden Vergrößerung mit einer Auflösung von 400 x 400 Bildpunkten, 2000 x 2000 Bildpunkten oder einer dazwischenliegenden Auflösung bereitgestellt, wobei $A_{min}$ zwischen 10 einschließlich und 50 einschließlich liegt.

Bevor die Auswahl des validen Bereichs des Blutausstriches 32 anhand der lokalen Häufigkeit und der lokalen mittleren Größe weiterbeschrieben wird, sei noch darauf hingewiesen, dass bei den nachfolgenden Ausführungsbeispielen exemplarisch davon ausgegangen wird, dass die Aufnahmen 36 den Blutausstrich 32 jeweils mit der gleichen lateralen Genauigkeit darstellen. Notwendig ist dies allerdings nicht. Eventuelle Unterschiede in der lateralen Genauigkeit bzw. dem Bildpunktabstand auf dem Blutausstrich 32 könnten auch bei der Bestimmung der Werte für die lokale Häufigkeit und die lokale mittlere Größe berücksichtigt werden.

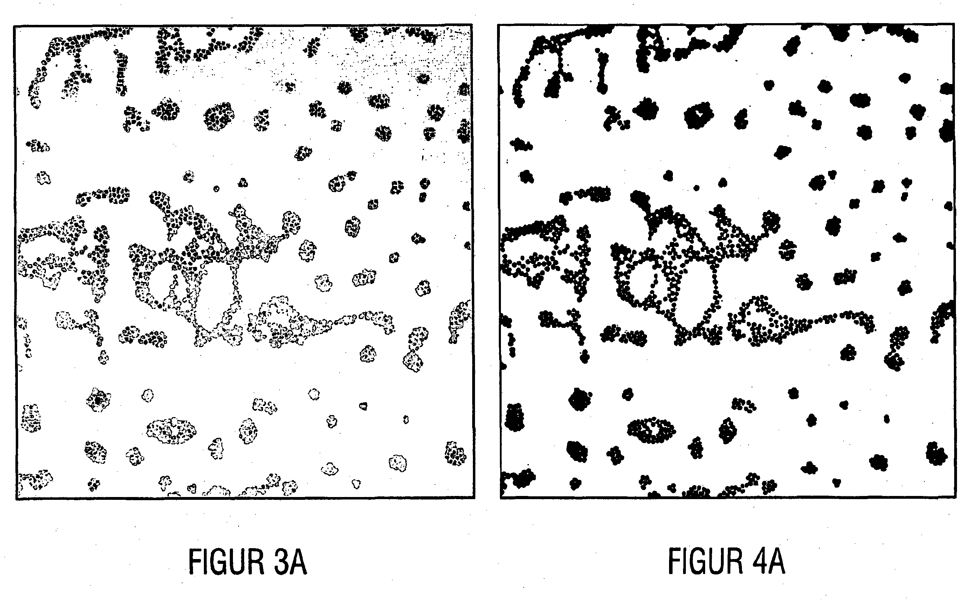

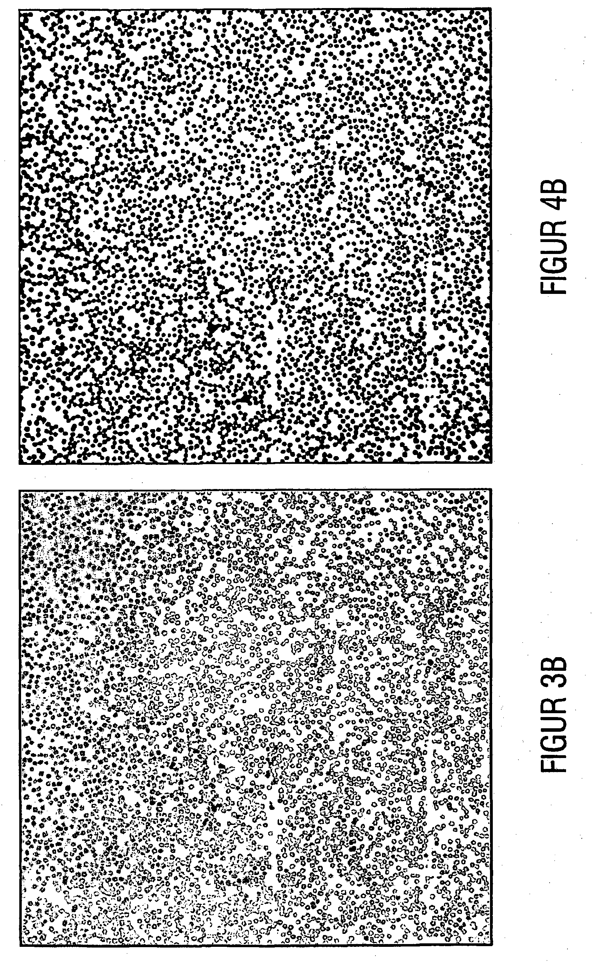

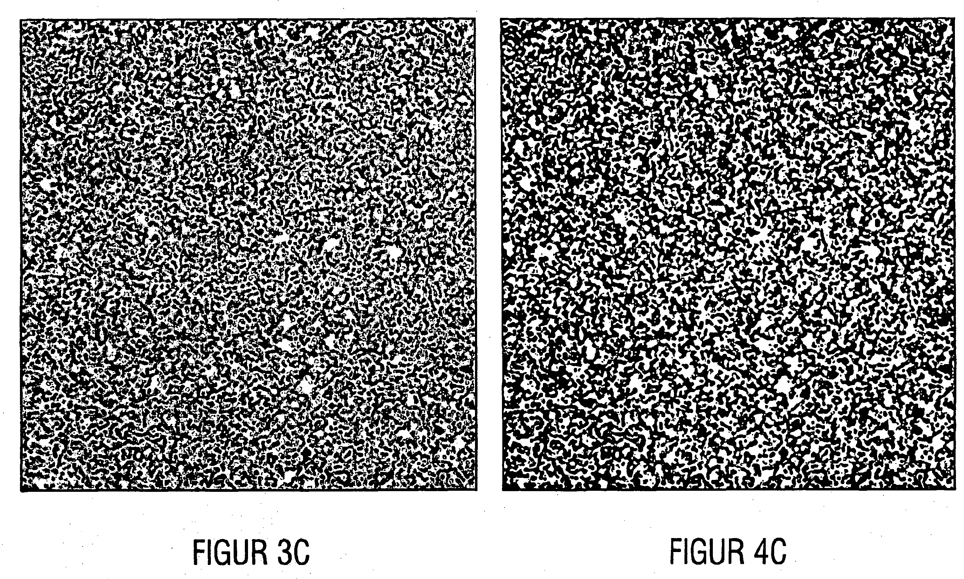

Wenn man die Wertepaare für die lokale Häufigkeit $N_i$ und die lokale mittlere Größe $A_i$ für die einzelnen Aufnahmen i in einer Ebene aufträgt, die durch eine Achse 46 für die lokale Häufigkeit N und eine Achse 48 für die lokale mittlere Größe A aufgespannt wird, dann liegen die entsprechenden Punkte, die in Fig. 2 exemplarisch mit dem Aufnahmeindex beschriftet sind und mit einem x angedeutet sind, typischer Weise entlang einer Spur 50. Aufgrund einer Eigenschaft des Vorgangs des Anfertigens eines Ausstriches sind die Punkte entlang der Spur 50 im Wesentlichen in der Reihenfolge angeordnet, die der Reihenfolge ihrer Anordnung entlang der Ausstrichrichtung 38 entspricht. Insbesondere beginnt die Spur an einem Ende mit Wertepaaren von Aufnahmen, bei denen, wie es bei 52 gezeigt ist, die Anzahl der Bildpunktcluster pro Aufnahme noch relativ gering ist, da sich die Blutzellen zu Klumpen zusammengezogen haben, so dass die Bildpunktcluster eine relativ große mittlere Größe aufweisen. Ein Beispiel für eine entsprechende Aufnahme 36 eines solchen Bereiches eines Bildausstriches und das beispielsweise entstehende Binärbild 42 sind in Fig. 3a bzw. 4a gezeigt. Sodann schließt sich ein Teil 54 an, innerhalb welchem die Blutzellen schön voneinander getrennt liegen. In diesem Teil 54 wächst für einige Zeit die Bildpunktclusteranzahl pro Aufnahme bei etwa gleichbleibender mittlerer Clustergröße. Irgendwann nimmt jedoch, wie es bei 56 gezeigt ist, die Anzahl der Bildpunktcluster bzw. Blutzellen in den Aufnahmen so zu, dass sich einige Bildpunktcluster bzw. Blutzelle berühren und zu größeren Clustern verschmelzen, und dieser Effekt bewirkt dann etwas später bei 58 sogar, dass sogar die Anzahl an Bildpunktclustem abnimmt, während die mittlere Größe der Bildpunktcluster zunimmt. Ein Beispiel für eine entsprechende Aufnahme 36 aus dem Bereich 58 und das beispielsweise daraus entstehende Binärbild 42 ist in Fig. 3c bzw. 4c gezeigt. Ein optimaler valider Bereich liegt in etwa zwischen den Bereichen 54 und 56 bzw. am Ende des Bereiches 54 und ein Beispiel hierfür bzw. eine entsprechende Aufnahme 36 eines solchen validen Bereiches und das beispielsweise daraus entstehende Binärbild 42 sind in Fig. 3b bzw. 4c gezeigt.

Der Auswähler 16 könnte nun ausgebildet sein, um den validen Bereich geometrisch aus einer Auswertung der Spur 50 zu ermitteln. Dazu könnte der Auswähler 16 beispielsweise die Positionen verwenden, an denen die einzelnen Aufnahmen angeordnet sind. Insbesondere könnte der Auswähler 16 aus der Spur 50 geometrisch das Ende des Bereiches 54 bzw. den Übergang zwischen den Bereichen 54 und 56 ermitteln, und als validen Bereich denjenigen auszuwählen, der der Aufnahme entspricht, die dieser Position der Spur 50 am nächsten kommt oder die Position, die sich zwischen den beiden Aufnahmen befindet, die sich zu dieser Position am nächsten befinden, wie z.B. mittels Interpolation zwischen den beiden Positionen.

Eine andere Vorgehensweise, die bei den nachfolgend beschriebenen Ausführungsbeispielen verwendet worden ist, besteht darin, dass der Auswähler 16 zu jedem Wertepaar $N_i$, $A_i$ ein Maß für die Eignung als valider Bereich bestimmt, wobei in Fig. 2 bei 60 exemplarisch die Änderung eines solchen Maßes über die Aufnahmenummer angezeigt ist. Der Auswähler 16 könnte als den validen Bereich denjenigen auswählen, bei dem das Maß extremal ist. In Fig. 2 ist dies exemplarisch bei 62 der Fall, welche Position in etwa der Aufnahme mit dem Aufnahmeindex 4 entspricht. Als validen Bereich könnte der Auswähler 16 auch eine Position zwischen zwei nächstgelegenen Aufnahmen des Extrempunktes wählen. Auf diese Vorgehensweise wird im Folgenden noch näher Bezug genommen, weshalb hier nicht näher darauf eingegangen wird.

Nachdem im Vorhergehenden Bezug nehmend auf Fig. 2 ein Beispiel für eine Lokalisierung eines validen Bereichs eines Blutausstriches eher qualitativ beschrieben worden ist, wird im Folgenden eine eher mathematischere Beschreibung verwendet. Dabei werden Beispiele für ein mögliches Maß für die Eignung als validen Bereich geliefert, das von der lokalen Häufigkeit und der lokalen mittleren Größe abhängt.

Wir betrachten einen Satz von M Bildern 36. Der Einfachheit halber sei angenommen, dass diese Bilder 36 anhand eines systematischen vollständigen Abtastens eines Objektträgers erhalten wurden. Um jedoch den Abtastvorgang zu beschleunigen, können willkürliche Heuristiken verwendet werden, um den Suchraum, wie er für die später zu beschreibende Auswertung angewendet wird, einzuschränken. Der Objektträger befindet sich bei den Aufnahmen beispielsweise auf einem Objekttisch, der seinen Inhalt (den Blutausstrich) der Optik des Mikroskops präsentiert. Ein vollständiges Abtasten wird erzielt, indem der Objekttisch bewegt wird, wobei ein Bild mit einem Vergrößerungsfaktor von beispielsweise 10 erhalten wird und die Position des Objekttischs gespeichert wird, bis der gesamte Objektträger in dem Aufnahmen festgehalten wurde. Der Objekttisch definiert beispielsweise ein globales Koordinatensystem mit einer x-, einer y- und einer z-Achse. Jedes Bild 36 des Blutausstrichs 32 entspricht der Position, an der es aufgenommen wurde, und umgekehrt. Die hier beschriebene Lokalisierung ist unabhängig von einer bestimmten Einfärbung. Jedoch sollte die Einfärbung einen ausreichend hohen Kontrast zwischen dem Hintergrund und den Zellen bewirken. Es können z.B. Blutausstriche verwendet werden, die unter Verwendung von MGG-Lösungen (MGG = May-Grünwald-Giemsa) nach Pappenheim eingefärbt wurden.

Bei einem derartigen Abtasten sei das *i-te* Bild mit $I_i$ bezeichnet, wohingegen seine entsprechende Position in Objektträgerkoordinaten durch $\mathbf{p}_i$ - $(x_i, y_i, z_i)$ gegeben ist. Die hochgestellte Beschriftung eines Bildes bezeichne den Farbraum, auf den es sich bezieht. Somit stellt das Bild $\mathbf{I}_i^{HSV}$ das Bild $I_i$ in dem HSV-Farbraum dar, und $\mathbf{I}_i^{(S)}$ beziehe sich auf seinen Sättigungskanal, wohingegen $\mathbf{I}_i^{(S)}(x, y)$ den Wert eines einzelnen Pixels bzw. Bildpunkts bei (x,y) in der referenzierten Ebene darstelle. Der Übersichtlichkeit halber wird die auf den Farbraum bezogene hochgestellte Notation weggelassen, wenn ein RGB-Bild betrachtet wird.

Für jedes Bild $I_i$ der Sequenz werden die folgenden Schritte durchgeführt:

- Umwandlung von $I_i$ in eine zylindrische HSV-Darstellung: $\mathbf{I}_i \rightarrow \mathbf{I}_i^{HSV}$

- Trennung 46 von Zellen von dem Hintergrund. Dafür wird ein Binarisierungsoperator B{.} auf den Sättigungskanal $I^{(S)}$ angewandt, was einen Schwellwert $\tau_i$:

$$\tau_i = B\left\{I_i^{(S)}\right\}$$

ergibt. Der Binarisierungsoperator $B\{.\}$ kann also beispielsweise einen Schwellwertvergleich mit dem Sättigungs-schwellwert $\tau_i$ umfassen, wobei Pixel (x,y) mit einem Sättigungswert $I^{(S)}$ von größer $\tau_i$ als Bildpunkte interpretiert werden, die die interessierenden Bildzellen darstellen, d.h. zum Vorder.grund gehören.

- Extraktion 44 der Anzahl $N_i$ von miteinander verbundenen Regionen, die durch eine Ansammlung mit einem Minimum von $A_{min}$ Pixeln gebildet sind, um die Erfassung kleiner Regionen, die durch Schmutz oder Rauschen auf dem Objektträger oder in dem optischen Pfad verursacht werden, zu verhindern. Eine Region $R_j$ wird durch alle benach-barten Pixel $p = (x, y)$ und $\tilde{p} = (\tilde{x}, \tilde{y})$ definiert, die zu dem Vordergrund gehören:

$$\tilde{N}_i = \left| \bigcup \left\{ R_j : \left| R_j \right| \geq A_{\min} \right\} \right|,$$

$$R_j = \left\{ p, \tilde{p} : I_i^{(S)}(p) \geq \tau_i \wedge I_i^{(S)}(\tilde{p}) \geq \tau_i \wedge \left\| p - \tilde{p} \right\| \leq 1 \right\}$$

Der Operator $|\cdot|$ gebe dabei die Anzahl der zu einer zusammenhängenden Region gehörenden Bildpunkte an. Der Operator $\|\cdot\|$ gebe den lateralen Abstand in Einheiten eines Pixelwiederholabstandes an.

- Extraktion 44 der Fläche $A_j$ der Regionen $R_j$ bzw. die Anzahl von Pixeln einer bestimmten Region $R_j$ ergitb sich zu

$$A_j = \left| \left\{ R_j : \left| R_j \right| \geq A_{\min} \right\} \right|, j-1,...,N_i$$

- Berechnung des durchschnittlichen Bereichs $\overline{A}_i$ aus den Bereichen $A_j$:

$$\overline{A}_i = \frac{1}{N_i} \sum_{j=1}^{N_i} A_j$$

Hier wurde der Mittelwert verendet, aber eine andere zentrale Tendenz ist ebenfalls möglich.

- Berechnung der Indikatorfunktion $\vartheta(i)$ als Maß für eine Eignung als valider Bereich. $\vartheta(i)$ ergibt einen charakteristi-schen Wert $i^*$ für die Position $p_{i^*}$ des Bildes i und ermöglicht die Bestimmung von Teilen der der validen Region des Blutausstrichs, die somit beispielsweise ein geeigneter Ausgangspunkt für die anschließende Bestimmung des WBC ist.

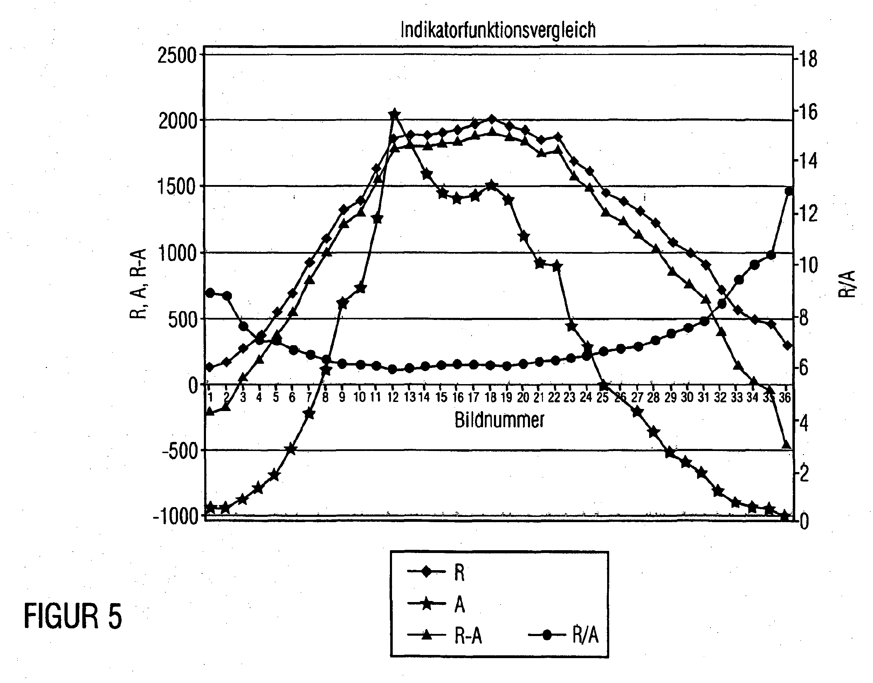

Im Folgenden werden zwei verschiedene Indikatorfunktionen $\vartheta(i)$, die auf $\overline{A}_i$ und $N_i$ beruhen, zum Zweck einer robusten Bestimmung des Indizes $i^*$ untersucht, der einer Position $p_i^*$ in der validen Region entspricht. Deshalb sind in Fig. 5 vier Funktionsgraphen eines beispielhaften Objektträgers dargestellt, die die Funktionen $\overline{A}_i$, $N_i$ und die Indikator-funktionen $\vartheta(i) = N_i - \overline{A}_i$ und $\vartheta(i) = \dfrac{N_i}{\overline{A}_i}$ zeigen. Die Position p* der validen Region ist dann durch das globale Maximum $i^*$ der Indikatorwerte $\vartheta(i)$ gegeben. Die Indikatorfunktion $\vartheta(i) = N_i - \overline{A}_i$ liefert gute Ergebnisse, berücksichtigt jedoch nicht ausreichend die durchschnittliche Clustergröße und führt zu einem globalen Maximum bei dem Bild 18, wohingegen der Experte die valide Region für die Bilder 11 bis 14 auswählte. Die Indikatorfunktion $\vartheta(i) = N_i / \overline{A}_i$, misst der Clustergröße mehr Bedeutung bei, weist ihr Maximum bei Bild 12 auf und ist somit eine zuverlässigere Gütefunktion, bei der der charakteristische Index $i^*$ durch das globale Maximum von $\vartheta(i)$ gegeben ist.
Die Position p* der validen Region ist dann durch das globale Maximum $i^*$ des Indikator-wertes $\vartheta(i)$ gegeben.

Die Position $p_i^*$, die dem Index $i^*$ entspricht, ist sicherlich ein Teil der validen Region des Blutausstrichs. Somit kann sie als Ausgangspunkt für eine anschließende Bestimmung des WBC verwendet werden.

Noch einmal in anderen Worten ausgedrückt können zur Ermittlung eines validen Bereiches M Aufnahmen in zehnfacher Vergrößerung des gesamten Blutausstriches in digitaler Form des Objektträgers erfasst werden, wobei die Position Pi der i-ten Aufnahme gespeichert wird. Anschließend werden die Aufnahmen mittels Bildverarbeituingsalgorithmen folgendermaßen ausgewertet:

Für jedes Bild $m_i$ der M Aufnahmen werden folgende Einzelschritte ausgeführt:

1. Transformation des RGB-Bildes in den HSV-Farbraum.

2. Automatisches Binarisierung wie zum Beispiel gemäß dem Artikel von Otsu et al., A Threshold Selction Method from Gray-Level Histograms, IEEE Transaction on Systems, MAN and Cybernetics, Nr. 1, Januar 1979, S. 62-64, wie z.B. anhand des Sättigungskanals (S-Kanal).

3. Bestimmung der Anzahl $N_i$ zusammenhängender Regionen, die im Binärbild als Vordergrund definiert sind.

4. Bestimmung der Fläche $F_j$, $j = 1 - N_i$, der o.g. zusammenhängenden Regionen (in Pixel).

5. Berechnung der durchschnittlichen Flächengröße $C_i$ aller Flächen $F_i$.

6. Berechnung der Differenz D($i$) zwischen der Anzahl der Regionen $N_i$ und der durchschnittlichen Flächengröße Ci: $D(i) = N_i - C_i$.

Eine Position P* des validen Bereiches des Blutausstriches ergibt sich aus dem globale Maximum i* der Funktion $D(i)$. Die dem Index i* zugeordnete Bildposition P*$_i$ ist sicher Teil des validen Bereiches und dient als Startpunkt für die anschließende Erstellung des Differentialblutbildes.

Im Rahmen einer automatischen Erstellung eines Differentialblutbildes ist die sichere Detektion des validen Bereichs unabdingbar. Die obigen Ausführungsbeispiele liefern die Grundlage für weitere Schritte bei einer automatischen Differentialblutbildanalyse. Bei der manuellen Erstellung muss dieser Bereich zunächst auf dem Objektträger lokalisiert werden, was zunächst einen höheren Zeitaufwand bedeutet. Weiterhin können sich hier Schwankungen ergeben. Beispielsweise wird nicht immer der optimale Bereich ausgewählt. Diese Schwankungen haben wiederum einen direkten Einfluss auf das Ergebnis der Differenzierung und damit unter Umständen auch auf die abschließende Diagnose, die sich auf diese Verteilung stützt. Die automatische Erkennung des validen Bereichs nach einem der obigen Ausführungsbeispiele bietet weiterhin noch die Möglichkeit schlecht oder unzureichend präparierte Ausstriche zu erkennen und somit die Qualität bei der Differentialblutbildanalyse zu steigern.

Um die Genauigkeit des dargestellten Lösungsansatzes zum Lokalisieren des validen Bereichs auf Blutausstrichen auszuwerten, wurden mehrere Bildsätze aufgenommen und analysiert. Die Bilder werden unter Verwendung des oben erwähnten HemaCAM-Systems aufgenommen. Abgesehen von einem wurden alle Objektträger mit einer Objektträgerherstellungsvorrichtung hergestellt und mit einer Färbevorrichtung gefärbt. Jeder der Bildsätze besteht aus 50 benachbarten Bildern, die entlang der Mittellinie der Längsrichtung des Blutausstrichs aufgenommen wurden, die den Großteil der linearen Ausdehnung eines Blutausstrichs - beginnend an dem Ende des Ausstrichs, an dem die Zellendichte gering ist

- darstellt. Diese Bildsätze wurden seitens eines erfahrenen Hämatologen ausgewertet und anhand des Algorithmus analysiert, um die Bilder zu finden, die die valide Region zeigen. Die Ergebnisse sind zum Vergleich in der Tabelle I aufgelistet.

Bei den meisten Bildsätzen fand der Experte mehr als ein Bild, das den validen Bereich des Blutausstriches zeigte, wohingegen der Algorithmus lediglich eine Bildnummer lieferte. Sogar bei Satz 22, bei dem ein manuell ausgestrichener und gefärbter Objektträger analysiert wurde und lediglich ein Bild den validen Bereich zeigt, stimmte der Algorithmus für jeden Bildsatz mit einer der Bildnummern, die der Experte auswählte, überein.

TABELLE 1

| Bildsatz | Hämatologe | Algorithmus |
|----------|------------|-------------|
| 1 | 9-12 | 9 |

(fortgesetzt)

| Bildsatz | Hämatologe | Algorithmus |
|---|---|---|
| 2 | 17-22 | 21 |
| 3 | 14-17 | 15 |
| 4 | 16-19 | 18 |
| 5 | 26, 27 | 27 |
| 6 | 13-15 | 13 |
| 7 | 17-19 | 19 |
| 8 | 17-19 | 19 |
| 9 | 16-20 | 20 |
| 10 | 17-20 | 19 |
| 11 | 16-20 | 20 |
| 12 | 15-19 | 18 |
| 13 | 8-10 | 10 |
| 14 | 16-20 | 19 |
| 15 | 24-27 | 25 |
| 16 | 14-17 | 14 |
| 17 | 12, 13 | 11 |
| 18 | 10-15 | 10 |
| 19 | 17-25 | 21 |
| 20 | 10-15 | 13 |
| 21 | 11-14 | 13 |
| 22 | 20 | 20 |

Die im Vorhergehenden erwähnten Ausführungsbeispiele könnten beispielsweise bei dem HemaCAM (eingetragenes Warenzeichen) -System der Fraunhofer-Gesellschaft eingesetzt werden. Dieses System wurde zur automatisierten Untersuchung von Blutausstrichen entwickelt, um den Arzt bei seiner WBC-basierten Diagnose zu unterstützen. Das System umfasst einen Axio Imager M1 von Zeiss mit einem automatisch höhenverstellbaren Tisch (z-Achse), einem Achroplan-Objektiv von Zeiss mit 10facher Vergrößerung, einen 1-fachen Tube Adapter, eine CCD-Kamera AVT Pike F-100C und eine LED-Beleuchtung. Außerdem umfasst das System einen für mehrere Objektträger ausgelegten Objekttisch von Märzhäuser, um den Objektträger unter dem Objektiv in zwei Richtungen zu bewegen (x- und y-Achse). Als Bestes schneidet die Software HemaCAM mit den Benutzeroberflächen-, Hardwaresteuerungs- und Bildverarbeitungsalgorithmen ab.

Mit anderen Worten liefert eine robuste Erfassung und präzise Segmentierung der weißen Blutzellen (Leukozyten) in gefärbten Blutausstrichen des peripheren Blutes die Basis für eine vollautomatische bildbasierte Erstellung des so genannten Differentialblutbildes im Zusammenhang mit der medizinischen Labordiagnostik (so genannte computergestützte Mikroskopie (CAM - computer assisted microscopy). Vor allem zur Lokalisierung der Blutzellen und insbesondere zur Segmentierung der Zellen ist es notwendig, die valide Region (den Arbeitsbereich) des Blutausstrichs zu erfassen, in der bzw. in dem die Zellen überwiegend getrennt sind und sich gegenseitig nicht beeinflussen. Die vorhergehenden Ausführungsbeispiele stellen einen Lösungsansatz zum Lokalisieren der validen Region auf gefärbten Blutausstrichen dar. Bei manchen Ausführungsbeispielen werden mehrere Bildaufnahmen des Blutausstrichs gemacht und wie folgt analysiert. Nach einer Binarisierung jeder Bildaufnahme unter Verwendung der anhand beispielsweise des Otsu-Algorithmus erhaltenen Schwelle werden die Regionen in den resultierenden Bildern analysiert. Die Anzahl der Regionen und ihre durchschnittliche Größe werden für jedes Bild gespeichert und miteinander verglichen. Das Bild, das den validen Bereich des Blutausstrichs zeigt, weist eine große Anzahl von Bereichen auf und eine geringe durchschnittliche Bereichsgröße. Die Auswertung des Verfahrens kann anhand mehrerer Bildsätze erfolgen, die von verschiedenen Blutausstrichen aufgenommen wurden. Jeder Bildsatz kann anhand eines der vorgestellten Algorithmen analysiert werden, und die Ergebnisse können durch einen erfahrenen Hämatologen überprüft werden. Die Ergebnisse der Auswertung weisen auf die Möglichkeiten des vorgestellten Lösungsansatzes hin.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer Blu-ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einem programmierbaren Computersystem derart zusammenwirken können oder zusammenwirken, dass das jeweilige Verfahren durchgeführt wird. Deshalb kann das digitale Speichermedium computerlesbar sein. Manche Ausführungsbeispiele gemäß der Erfindung umfassen also einen Datenträger, der elektronisch lesbare Steuersignale aufweist, die in der Lage sind, mit einem programmierbaren Computersystem derart zusammenzuwirken, dass eines der hierin beschriebenen Verfahren durchgeführt wird.

Allgemein können Ausführungsbeispiele der vorliegenden Erfindung als Computerprogrammprodukt mit einem Programmcode implementiert sein, wobei der Programmcode dahin gehend wirksam ist, eines der Verfahren durchzuführen, wenn das Computerprogrammprodukt auf einem Computer abläuft. Der Programmcode kann beispielsweise auch auf einem maschinenlesbaren Träger gespeichert sein.

Andere Ausführungsbeispiele umfassen das Computerprogramm zum Durchführen eines der hierin beschriebenen Verfahren, wobei das Computerprogramm auf einem maschinenlesbaren Träger gespeichert ist.

Mit anderen Worten ist ein Ausführungsbeispiel des erfindungsgemäßen Verfahrens somit ein Computerprogramm, das einen Programmcode zum Durchführen eines der hierin beschriebenen Verfahren aufweist, wenn das Computerprogramm auf einem Computer abläuft. Ein weiteres Ausführungsbeispiel der erfindungsgemäßen Verfahren ist somit ein Datenträger (oder ein digitales Speichermedium oder ein computerlesbares Medium), auf dem das Computerprogramm zum Durchführen eines der hierin beschriebenen Verfahren aufgezeichnet ist.

Ein weiteres Ausführungsbeispiel des erfindungsgemäßen Verfahrens ist somit ein Datenstrom oder eine Sequenz von Signalen, der bzw. die das Computerprogramm zum Durchführen eines der hierin beschriebenen Verfahren darstellt bzw. darstellen. Der Datenstrom oder die Sequenz von Signalen kann bzw. können beispielsweise dahingehend konfiguriert sein, über eine Datenkommunikationsverbindung, beispielsweise über das Internet, transferiert zu werden.

Ein weiteres Ausführungsbeispiel umfasst eine Verarbeitungseinrichtung, beispielsweise einen Computer oder ein programmierbares Logikbauelement, die dahin gehend konfiguriert oder angepasst ist, eines der hierin beschriebenen Verfahren durchzuführen.

Ein weiteres Ausführungsbeispiel umfasst einen Computer, auf dem das Computerprogramm zum Durchführen eines der hierin beschriebenen Verfahren installiert ist.

Bei manchen Ausführungsbeispielen kann ein programmierbares Logikbauelement (beispielsweise ein feldprogrammierbares Gatterarray, ein FPGA) dazu verwendet werden, manche oder alle Funktionalitäten der hierin beschriebenen Verfahren durchzuführen. Bei manchen Ausführungsbeispielen kann ein feldprogrammierbares Gatterarray mit einem Mikroprozessor zusammenwirken, um eines der hierin beschriebenen Verfahren durchzuführen. Allgemein werden die Verfahren bei einigen Ausführungsbeispielen seitens einer beliebigen Hardwarevorrichtung durchgeführt. Diese kann eine universell einsetzbare Hardware wie ein Computerprozessor (CPU) sein oder für das Verfahren spezifische Hardware, wie beispielsweise ein ASIC.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

Ein erfindungsgemäß codiertes Signal, wie beispielsweise ein Audio- oder ein Videosignal, kann auf einem digitalen Speichermedium gespeichert sein oder kann auf einem Übertragungsmedium wie beispielsweise einem drahtlosen Übertragungsmedium oder einem verdrahteten Übertragungsmedium, z.B. dem Internet, übertragen werden

**Patentansprüche**

**1.** Vorrichtung zur Lokalisierung eines validen Bereiches eines Blutausstriches, mit

einer Einrichtung (12) zum Bereitstellen zumindest einer Aufnahme (36) des Blutausstriches (32);

einer Einrichtung (14) zum Klassifizieren von Bildpunkten der zumindest einen Aufnahme (36) zumindest in erste Bildpunkte, die Blutzellen darstellen, und zweite Bildpunkte, die die Blutzellen nicht darstellen;

einer Einrichtung (16) zum Auswählen eines Bereichs des Blutausstriches (32) als den validen Bereich,

**dadurch gekennzeichnet, dass** die Einrichtung (16) zum Auswählen eines Bereichs des Blutausstriches (32) als den validen Bereich ausgebildet ist, die Auswahl basierend auf einer lokalen Häufigkeit ($N_i$) von zusammenhängenden Bildpunktclustern von zumindest $A_{min}$ ersten Bildpunkten, wobei $A_{min}$ ein Mindestschwellwert für eine Anzahl von ersten Bildpunkten eines Bildpunktclusters ist, und einer lokalen mittleren Größe ($A_i$) der Bildpunktcluster für lateral verteilte Bereiche des Blutausstriches (32) vorzunehmen.

2. Vorrichtung gemäß Anspruch 1, bei der die Einrichtung (16) zum Auswählen ausgebildet ist, um auf Basis der lokalen Häufigkeit ($N_i$) und der lokalen mittleren Größe ($A_i$) der Bildpunktcluster für die lateral verteilten Bereiche des Blutausstriches (32) ein lokal variierendes Maß (60) für eine Eignung als valider Bereich zu bestimmen und die Auswahl so zu treffen, dass derjenige Bereich unter den lateral verteilten Bereichen als der valide Bereich ausgewählt wird, bei dem das lateral variierende Maß extremal ist.

3. Vorrichtung gemäß Anspruch 2, bei der die Einrichtung (16) zum Auswählen so ausgebildet ist, dass das lateral variierende Maß von der lokalen Häufigkeit und der lokalen mittleren Größe für die lateral verteilten Bereiche gemäß einer Funktion abhängt, die entweder streng monoton fallend für die lokale Häufigkeit und streng monoton steigend für die lokale mittlere Größe oder streng monoton steigend für die lokale Häufigkeit und streng monoton fallend für die lokale mittlere Größe ist.

4. Vorrichtung gemäß Anspruch 2, bei die Einrichtung (16) zum Auswählen so ausgebildet ist, dass dieselbe das lateral variierende Maß für jeden der lateral verteilten Bereiche auf Basis eines Quotienten aus oder einer Differenz von der lokalen Häufigkeit und der lokalen mittleren Größe für den jeweiligen der lokal verteilten Bereiche bestimmt.

5. Vorrichtung gemäß Anspruch 2, bei der die Einrichtung (16) zum Auswählen so ausgebildet ist, dass sie das lateral variierende Maß für jeden der lateral verteilten Bereiche zu $k(N-A)$ bestimmt, wobei k eine Konstante ungleich Null, N die Anzahl der Bildpunktcluster innerhalb des jeweiligen Bereiches der lateral verteilten Bereiche und A die mittlere Anzahl von Bildpunkten der Bildpunktcluster innerhalb des jeweiligen Bereiches der lateral verteilten Bereiche ist, wobei die lateral verteilten Bereiche gleich groß sind.

6. Vorrichtung gemäß Anspruch 2, bei der die Einrichtung (16) zum Auswählen so ausgebildet ist, dass sie das lateral variierende Maß für jeden der lateral verteilten Bereiche zu $k(N/A)$ bestimmt, wobei k eine Konstante ungleich Null, N die Anzahl der Bildpunktcluster innerhalb des jeweiligen Bereiches der lateral verteilten Bereiche und A die mittlere Anzahl von Bildpunkten der Bildpunktcluster innerhalb des jeweiligen Bereiches der lateral verteilten Bereiche ist, wobei die lateral verteilten Bereiche gleich groß sind.

7. Vorrichtung gemäß einem der vorhergehenden Ansprüche, bei der die Einrichtung (12) zum Bereitstellen ausgebildet ist, um mehrere Aufnahmen (36) des Blutausstriches (32) bereitzustellen, die zueinander lateral versetzte Teile des Blutausstriches (32) darstellen.

8. Vorrichtung gemäß Anspruch 7, bei der die Einrichtung (16) zum Auswählen so ausgebildet ist, dass jede Aufnahme (36) einem unterschiedlichen der lateral verteilten Bereiche zugeordnet ist, und ausgebildet ist, um für jede Aufnahme die lokale Häufigkeit basierend auf einer Anzahl der zusammenhängenden Bildpunktcluster in der jeweiligen Aufnahme und die lokale mittlere Größe basierend auf der Anzahl der Bildpunkte pro Bildpunktcluster zu bestimmen.

9. Vorrichtung gemäß Anspruch 7 oder 8, bei der die Einrichtung (12) zum Bereitstellen ausgebildet ist, um die Aufnahmen mit einer 10-fachen, einer 20-fachen oder einer dazwischen liegenden Vergrößerung mit einer Auflösung von 400 x 400 Bildpunkten, 2000 x 2000 Bildpunkten oder einer dazwischenliegenden Auflösung bereitzustellen, wobei die Einrichtung (16) zum Auswählen derart ausgebildet ist, dass $A_{min}$ zwischen 10 einschließlich und 50 einschließlich liegt.

10. Vorrichtung gemäß einem der vorhergehenden Ansprüche, bei der die Einrichtung (14) zum Klassifizieren ausgebildet ist, um die Klassifikation basierend auf einer Aufteilung eines Farbraums in zumindest einen ersten Teil, der den ersten Bildpunkten zugeordnet ist, und einen zweiten Teil, der den zweiten Bildpunkten zugeordnet ist, durchzuführen.

**11.** Vorrichtung gemäß einem der vorhergehenden Ansprüche, bei der die Einrichtung (14) zum Klassifizieren derart ausgebildet ist, dass dieselbe zwischen Bildpunkten, die rote Blutzellen darstellen, und Bildpunkten, die weiße Blutzellen darstellen, unterscheidet, und nur diejenigen Bildpunkte unter denselben als die ersten Bildpunkte klassifiziert, die die roten Blutzellen darstellen.

**12.** Computergestütztes Verfahren zur Lokalisierung eines validen Bereiches eines Blutausstriches, mit
Bereitstellen zumindest einer Aufnahme (36) des Blutausstriches (32);
Klassifizieren von Bildpunkten der zumindest einen Aufnahme (36) zumindest in erste Bildpunkte, die Blutzellen darstellen, und zweite Bildpunkte, die die Blutzellen nicht darstellen;
Auswählen eines Bereichs des Blutausstriches (32) als den validen Bereich,
**dadurch gekennzeichnet, dass** die Auswahl basierend auf einer lokalen Häufigkeit von zusammenhängenden Bildpunktclustern von zumindest $A_{min}$ ersten Bildpunkten, wobei $A_{min}$ einen Mindestschwellwert für eine Anzahl von ersten Bildpunkten eines Bildpunktclusters ist, und einer lokalen mittleren Größe der Bildpunktcluster für lateral verteilte Bereiche des Blutausstriches (32) vorgenommen wird.

**13.** Computerprogramm zur Durchführung eines Verfahrens gemäß Anspruch 12, wenn das Computerprogramm von einem Prozessor ausgeführt wird.

## Claims

**1.** An apparatus for localizing a valid area of a blood smear, comprising
a means (12) for providing at least one picture (36) of the blood smear (32);
a means (14) for classifying pixels of the at least one picture (36) at least into first pixels, which represent blood cells, and second pixels, which do not represent the blood cells;
a means (16) for selecting an area of the blood smear (32) as the valid area,
**characterized in that** the means (16) for selecting an area of the blood smear (32) as the valid area is configured to perform the selection on the basis of a local frequency (Ni) of contiguous pixel clusters of at least $A_{min}$ first pixels, $A_{min}$ being a minimum threshold value of a number of first pixels of a pixel cluster, and of a local average size (Ai) of the pixel clusters for laterally distributed areas of the blood smear (32).

**2.** The apparatus as claimed in claim 1, wherein the means (16) for selecting is configured to select, on the basis of the local frequency (Ni) and the local average size (Ai) of the pixel clusters, a locally varying measure (60) of suitability as a valid area for the laterally distributed areas of the blood smear (32), and to make the selection such that that area wherein the laterally varying measure is extremal will be selected as the valid area among the laterally distributed areas.

**3.** The apparatus as claimed in claim 2, wherein the means (16) for selecting is configured such that the laterally varying measure depends on the local frequency and on the local average size for the laterally distributed areas in accordance with a function that is either strictly monotonically decreasing for the local frequency and strictly monotonically increasing for the local average size, or is strictly monotonically increasing for the local frequency and strictly monotonically decreasing for the local average size.

**4.** The apparatus as claimed in claim 2, wherein the means (16) for selecting is configured such that it determines the laterally varying measure for each of the laterally distributed areas on the basis of a quotient of or a difference between the local frequency and the local average size for the respective one of the locally distributed areas.

**5.** The apparatus as claimed in claim 2, wherein the means (16) for selecting is configured such that it determines the laterally varying measure for each of the laterally distributed areas to be k(N-A), wherein k is a constant different from zero, N is the number of pixel clusters within the respective area of the laterally distributed areas, and A is the average number of pixels of the pixel clusters within the respective area of the laterally distributed areas, the laterally distributed areas being equal in size.

**6.** The apparatus as claimed in claim 2, wherein the means (16) for selecting is configured such that it determines the laterally varying measure for each of the laterally distributed areas to be k(N/A), wherein k is a constant different from zero, N is the number of pixel clusters within the respective area of the laterally distributed areas, and A is the average number of pixels of the pixel clusters within the respective area of the laterally distributed areas, the laterally distributed areas being equal in size.

7. The apparatus as claimed in any of the previous claims, wherein the means (12) for providing is configured to provide several pictures (36) of the blood smear (32), which represent portions of the blood smear (32) that are laterally offset from one another.

8. The apparatus as claimed in claim 7, wherein the means (16) for selecting is configured such that each picture (36) is associated with a different one of the laterally distributed areas, and is configured to determine, for each picture, the local frequency on the basis of a number of the contiguous pixel clusters in the respective picture, and the local average size on the basis of the number of pixels per pixel cluster.

9. The apparatus as claimed in claims 7 or 8, wherein the means (12) for providing is configured to provide the pictures with a 10-fold or a 20-fold magnification or any magnification in between at a resolution of 400 x 400 pixels, 2000 x 2000 pixels or any resolution in between, the means (16) for selecting being configured such that $A_{min}$ ranges from 10, inclusively, to 50, inclusively.

10. The apparatus as claimed in any of the previous claims, wherein the means (14) for classifying is configured to perform the classification on the basis of a subdivision of a color space into at least a first portion, which is associated with the first pixels, and a second portion, which is associated with the second pixels.

11. The apparatus as claimed in any of the previous claims, wherein the means (14) for classifying is configured such that it distinguishes between pixels representing red blood cells and pixels representing white blood cells, and classifies as the first pixels only those pixels among same which represent the red blood cells.

12. A computer-aided method of localizing a valid area of a blood smear, comprising
providing at least one picture (36) of the blood smear (32);
classifying pixels of the at least one picture (36) at least into first pixels, which represent blood cells, and second pixels, which do not represent the blood cells;
selecting an area of the blood smear (32) as the valid area,
**characterized in that** the selection is performed on the basis of a local frequency of contiguous pixel clusters of at least $A_{min}$ first pixels, $A_{min}$ being a minimum threshold value of a number of first pixels of a pixel cluster, and of a local average size of the pixel clusters for laterally distributed areas of the blood smear (32).

13. A computer program for performing a method as claimed in claim 12, when the computer program is performed by a processor.

**Revendications**

1. Dispositif de localisation d'une zone saine d'un frottis sanguin, avec
un moyen (12) destiné à préparer au moins une prise de vue (36) du frottis sanguin (32);
un moyen (14) destiné à classifier des pixels de l'au moins une prise de vue (36) au moins en premiers pixels qui représentent des cellules sanguines, et en deuxièmes pixels qui ne représentent pas les cellules sanguines;
un moyen (16) destiné à sélectionner une zone du frottis sanguin (32) comme zone saine,
**caractérisé par le fait que** le moyen (16) destiné à sélectionner une zone du frottis sanguin (32) comme zone saine est réalisé pour procéder à la sélection sur base d'une fréquence locale ($N_i$) de groupes de pixels contigus d'au moins $A_{min}$ premiers pixels, $A_{min}$ étant une valeur de seuil minimum pour un nombre de premiers pixels d'un groupe de pixels, et d'une grandeur moyenne locale ($A_i$) des groupes de pixels pour des zones réparties latéralement du frottis sanguin (32).

2. Dispositif selon la revendication 1, dans lequel le moyen (16) destiné à sélectionner est réalisé pour déterminer, sur base de la fréquence locale ($N_i$) et de la grandeur moyenne locale ($A_i$) des groupes de pixels, pour les zones réparties latéralement du frottis sanguin (32), une mesure variable localement (60) d'aptitude comme zone saine et pour effectuer le choix de sorte que soit sélectionnée comme zone saine la zone parmi les zones réparties latéralement dans laquelle la mesure variable latéralement est extrême.

3. Dispositif selon la revendication 2, dans lequel le moyen (16) destiné à sélectionner est réalisé de sorte que la mesure variable latéralement soit fonction de la fréquence locale et de la grandeur moyenne locale pour les zones distribuées latéralement selon une fonction qui est soit décroissante de manière strictement monotone pour la fréquence locale et croissante de manière strictement monotone pour la grandeur moyenne locale ou croissante de manière strictement monotone pour la fréquence locale et décroissante de manière strictement monotone pour la grandeur moyenne locale.

4. Dispositif selon la revendication 2, dans lequel le moyen (16) destiné à sélectionner est réalisé de sorte qu'il détermine la mesure variable latéralement pour chacune des zones distribuées latéralement sur base d'un quotient de ou d'une différence entre la fréquence locale et la grandeur moyenne locale pour chacune des zones distribuées localement.

5. Dispositif selon la revendication 2, dans lequel le moyen (16) destiné à sélectionner est réalisé de sorte qu'il détermine la mesure variable latéralement pour chacune des zones réparties latéralement à k(N-A), où k est une constante différente de zéro, N est le nombre de groupes de pixels dans la zone respective des zones réparties latéralement et A est le nombre moyen de pixels des groupes de pixels dans la zone respective des zones réparties latéralement, où les zones réparties latéralement sont de même grandeur.

6. Dispositif selon la revendication 2, dans lequel le moyen (16) destiné à sélectionner est réalisé de sorte qu'il détermine la mesure variable latéralement pour chacune des zones réparties latéralement à k(N/A), où k est une constante différente de zéro, N est le nombre de groupes de pixels dans la zone respective des zones divisées latéralement et A est le nombre moyen de pixels des groupes de pixels dans la zone respective des zones réparties latéralement, où les zones réparties latéralement sont de même grandeur.

7. Dispositif selon l'une des revendications précédentes, dans lequel le moyen (12) destiné à préparer est réalisé pour préparer plusieurs prises de vue (36) du frottis sanguin (32) qui représentent des parties du frottis sanguin (32) décalées latéralement l'une de l'autre.

8. Dispositif selon la revendication 7, dans lequel le moyen (16) destiné à sélectionner est réalisé de sorte que chaque prise de vue (36) soit associée à l'une différente des zones réparties latéralement, et est réalisé pour déterminer, pour chaque prise de vue, la fréquence locale sur base d'un nombre de groupes de pixels contigus dans la prise de vue respective et la grandeur moyenne locale sur base du nombre de pixels par groupe de pixels.

9. Dispositif selon la revendication 7 ou 8, dans lequel le moyen (12) destiné à préparer est configuré pour préparer des prises de vue avec un agrandissement de 10 fois, de 20 fois ou un agrandissement intermédiaire avec une résolution de 400 x 400 pixels, de 2000 x 2000 pixels ou une résolution intermédiaire, dans lequel le moyen (16) destiné à sélectionner est réalisé de sorte que $A_{min}$ soit compris entre 10 et 50, tous deux inclus.

10. Dispositif selon l'une des revendications précédentes, dans lequel le moyen (14) destiné à classifier est réalisé pour effectuer la classification sur base d'une division d'un espace de couleurs en au moins une première partie associée aux premiers pixels et une deuxième partie associée aux deuxièmes pixels.

11. Dispositif selon l'une des revendications précédentes, dans lequel le moyen (14) destiné à classifier est configuré de sorte qu'il distingue entre les pixels qui représentent des globules rouges et les pixels qui représentent des globules blancs, et qu'il ne classifie comme premiers pixels que les pixels parmi ces derniers qui représentent les globules rouges.

12. Procédé assisté par ordinateur pour la localisation d'une zone saine d'un frottis sanguin, avec le fait de
préparer au moins une prise de vue (36) du frottis sanguin (32);
classifier les pixels de l'au moins une prise de vue (36) au moins en premiers pixels qui représentent des cellules sanguines et en deuxièmes pixels qui ne représentent pas les cellules sanguines;
sélectionner une zone du frottis sanguin (32) comme zone saine,
**caractérisé par le fait qu'**il est procédé à la sélection sur base d'une fréquence locale de groupes de pixels contigus d'au moins $A_{min}$ premiers pixels, $A_{min}$ étant une valeur de seuil minimale pour un nombre de premiers pixels d'un groupe de pixels, et d'une grandeur moyenne locale des groupes de pixels pour les zones réparties latéralement du frottis sanguin (32).

13. Programme d'ordinateur pour réaliser un procédé selon la revendication 12 lorsque le programme d'ordinateur est exécuté par un processeur.

EP 2 440 920 B1

10

18

20

| Aufnahmebereitsteller | | Klassifizierer | | Auswähler |

12

14

16

FIGUR 1

FIGUR 2

FIGUR 4A

FIGUR 3A

FIGUR 4B

FIGUR 3B

FIGUR 4C

FIGUR 3C

**FIGUR 6A**

**FIGUR 6B**

**FIGUR 6C**

15 - 40°

**FIGUR 6D**

Indikatorfunktionsvergleich

FIGUR 5

Legend:
R
A
R-A    R/A

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

*   DE 10353785 B4

### In der Beschreibung aufgeführte Nicht-Patentliteratur

*   **THEML, H. ; H. OIEM ; T. HAFERLACH.** Taschen-atlas der Hämatologie. Thiem Verlag, 2002, 16-18
*   **MUES-HINTERWÄLLER, S. ; KUZIELA, H. ; GROBE M. ; WITTENBERG, T.** Detektion und berandungsgenaue Segmentierung von Erythrozyten. *Bildverarbeitung für die Medizin,* 2005, 380-384
*   **THEML, H. ; H. DIEM ; T. HAFERLACH.** Taschen-atlas der Hämatologie. Thieme Verlag, 2002
*   **VON OTSU et al.** *A Threshold Selction Method from Gray-Level Histograms, IEEE Transaction on Systems, MAN and Cybernetics,* Januar 1999, 62-64